(19) Europäisches Patentamt
European Patent Office
Office européen des brevets

(11) **EP 3 081 857 B1**

(12) **FASCICULE DE BREVET EUROPEEN**

(45) Date de publication et mention
de la délivrance du brevet:
**08.11.2017 Bulletin 2017/45**

(51) Int Cl.:
***F21V 23/04*** *(2006.01)*     ***F21V 21/40*** *(2006.01)*

(21) Numéro de dépôt: **16165342.3**

(22) Date de dépôt: **14.04.2016**

(54) **DISPOSITIF D'ECLAIRAGE MEDICAL**

MEDIZINISCHE BELEUCHTUNGSVORRICHTUNG

MEDICAL LIGHTING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.04.2015 FR 1553336**

(43) Date de publication de la demande:
**19.10.2016 Bulletin 2016/42**

(73) Titulaire: **Steris**
**33185 Le Haillan (FR)**

(72) Inventeurs:
• **BESNARD, Mathieu**
**33160 SAINT MEDARD EN JALLES (FR)**
• **L'HEGARAT, Jean-Marie**
**33160 SAINT AUBIN DE MEDOC (FR)**

(74) Mandataire: **Robert, Mathias et al**
**Ernest Gutmann - Yves Plasseraud**
**S.A.S.**
**3, rue Auber**
**75009 Paris (FR)**

(56) Documents cités:
**EP-A1- 2 065 634**     **US-A1- 2006 109 650**

**Description**

La présente invention concerne un dispositif d'éclairage médical, notamment pour un bloc opératoire.

Un tel dispositif d'éclairage comporte classiquement un bras articulé dont une extrémité est fixe et dont une extrémité opposée mobile est équipée d'un module d'éclairage.

Le module d'éclairage peut être déplacé par un chirurgien, de manière à orienter le faisceau lumineux vers une zone à éclairer d'un patient.

Les besoins en quantité de lumière, en forme et en taille de la tâche lumineuse générée par le dispositif d'éclairage peuvent varier, notamment en fonction du type d'intervention pratiquée.

Le brevet EP 1 722 157 divulgue un dispositif d'éclairage médical, notamment pour un bloc opératoire, comportant une tête ou coupole d'éclairage comprenant un module central et des modules périphériques entourant le module central, chaque module comprenant des sources lumineuses formées par des diodes électroluminescentes. Les sources lumineuses des différents modules sont réparties en trois zones concentriques, l'intensité de chaque zone pouvant être contrôlée indépendamment. Il est ainsi possible d'ajuster la dimension et l'intensité de la tâche lumineuse générée par la tête d'éclairage. Le document EP 2.065.634 divulgue un dispositif d'éclairage médical. L'invention propose une alternative aux dispositifs précités permettant d'ajuster la luminosité et/ou la dimension de la tâche lumineuse produite, de manière simple, efficace et économique.

A cet effet, elle propose un dispositif d'éclairage médical, selon la revendication 1. De cette manière, la tête d'éclairage est apte à former une première tâche lumineuse dans le premier mode d'éclairage et une deuxième tâche lumineuse dans le deuxième mode d'éclairage, la deuxième tâche lumineuse présentant une dimension et/ou intensité plus importante que la première tâche lumineuse.

Il est par exemple possible de définir la dimension de la tâche lumineuse par la dimension de la zone dont l'intensité lumineuse est supérieure à 10% de l'intensité maximale de la tâche. Cette dimension peut être le diamètre de la tâche si cette dernière présente une forme circulaire.

Le dispositif peut comprendre au moins une troisième source lumineuse, décalée latéralement de la première source lumineuse par rapport à la direction d'éclairage de la première source lumineuse, les moyens de commande étant aptes à allumer les première, deuxième et troisième sources lumineuse dans un troisième mode d'éclairage.

De préférence, le dispositif peut comprendre au moins une quatrième source lumineuse, décalée latéralement de la première source lumineuse par rapport à la direction d'éclairage de la première source lumineuse, les moyens de commande étant aptes à allumer les première, deuxième, troisième et quatrième sources lumineuses dans un quatrième mode d'éclairage.

La première source lumineuse peut former une source lumineuse centrale, les deuxième, troisième et/ou quatrième sources lumineuses formant des sources lumineuses périphériques régulièrement réparties autour de la première source lumineuse.

Les différentes sources lumineuses peuvent être aptes à générer respectivement des tâches lumineuses superposées, au moins en partie, à une distance focale déterminée.

Il est ainsi possible de faire varier l'intensité de la zone lumineuse dans laquelle les différentes tâches lumineuses se superposent.

Selon l'invention, la tête d'éclairage peut comporter un corps dans lequel sont montées les sources lumineuses, une poignée s'étendant selon un axe, montée sur le corps et mobile en rotation par rapport au corps autour de l'axe de ladite poignée, des moyens de commande situés dans le corps et aptes à commander l'arrêt ou l'allumage des sources lumineuses, le passage d'un mode d'éclairage à l'autre étant obtenu par pivotement de la poignée autour de son axe.Selon l'invention, la course angulaire de la poignée est limitée par deux butées définissant deux positions extrêmes opposées de la poignée, le dispositif comportant en outre des moyens de détection aptes à détecter respectivement une première et une seconde positions angulaires de détection de la poignée, décalées angulairement respectivement par rapport auxdites positions extrêmes.

De cette manière, on garantit que les première et seconde positions angulaires sont correctement détectées par les moyens de détection avant que la poignée arrive en butée lors de son pivotement.

Le dispositif peut également comporter des moyens d'indexage, aptes à exercer un couple résistant sur la poignée, dans au moins une position angulaire déterminée de la poignée, de façon à indexer ladite position déterminée.

Dans ce cas, les moyens d'indexage peuvent être aptes à indexer des positions situées entre les première et seconde positions angulaires de détection par exemple juste avant lesdites positions de détection, et/ou une position médiane de la poignée située entre lesdites positions angulaires de détection. L'indexation en position médiane et celles juste avant les positions de détection permettent de réduire le risque de contrôle intempestif de l'éclairage lors du positionnement de la tête d'éclairage dans l'espace par un opérateur.

Par ailleurs, les moyens de détection peuvent comporter au moins une première partie couplée en rotation par rapport à la poignée et au moins une seconde partie couplée en rotation par rapport au corps et apte à fournir aux moyens de commande un signal qui est représentatif de la position angulaire la poignée par rapport au corps, la seconde partie étant apte à détecter la position angulaire de la première partie sans contact, ou inversement.

Les moyens de détection peuvent être du type sans contact, de façon à augmenter la fiabilité du dispositif. En effet, de tels moyens ne risquent pas d'être dégradés par frottement de pièces pivotant ou pouvant vibrer l'une par rapport à l'autre.

En particulier, les moyens de détection peuvent comporter au moins une première partie couplée en rotation par rapport à la poignée et au moins une seconde partie couplée en rotation par rapport au corps, la seconde partie étant apte à détecter la position angulaire de la première partie sans contact, ou inversement.

Dans ce cas, les moyens de détection peuvent comporter au moins une fourche optique comprenant un émetteur lumineux et un récepteur situé en regard de l'émetteur et apte à détecter un signal lumineux émis par ledit émetteur, les moyens de détection comportant en outre un obstacle apte à venir s'intercaler, en fonction de la position angulaire de la poignée, entre l'émetteur et le récepteur de la fourche optique. En particulier, les moyens de détection peuvent comporter au moins deux fourches optiques aptes à détecter deux positions angulaires différentes de la poignée.

En variante, les moyens de détection peuvent comporter au moins un capteur à effet Hall au moins un capteur inductif ou au moins un capteur capacitif. De tels capteurs sont également du type sans contact.

En outre, le dispositif peut comporter au moins un indicateur lumineux, tel par exemple qu'une diode électroluminescente, logée dans le corps et apte à fournir une information sur un mode de fonctionnement du dispositif, au moins une surface d'affichage visible depuis l'extérieur de la tête d'éclairage, au moins un guide lumineux comprenant une première extrémité située en regard et à distance de l'indicateur lumineux et une seconde extrémité située au niveau de la surface d'affichage, la lumière générée par ledit indicateur lumineux étant apte à être guidée jusqu'à la surface d'affichage par ledit guide lumineux.

Le dispositif peut également comporter des moyens de diffusion de la lumière issue de la source lumineuse, lesdits moyens de diffusion comportant au moins deux lentilles mobiles l'une par rapport à l'autre et comportant chacune une surface plane et une surface ondulée présentant des creux et des saillies, lesdites surfaces ondulées des lentilles étant agencées en regard l'une de l'autre, de façon à faire converger ou diverger les rayons lumineux qui les traversent en fonction de leurs positions respectives, le déplacement de l'une au moins des lentilles par rapport à l'autre étant effectué par pivotement de la poignée.

L'invention sera mieux comprise et d'autres détails, caractéristiques et avantages de l'invention apparaîtront à la lecture de la description suivante faite à titre d'exemple non limitatif en référence aux dessins annexés dans lesquels :

- la figure 1 est une vue en perspective d'un dispositif d'éclairage selon une forme de réalisation de l'invention,
- la figure 2 est une vue éclatée, en perspective, d'une partie du dispositif de la figure 1,
- la figure 3 est une vue en perspective, de dessous, d'une partie du dispositif,
- les figures 4 et 5 sont des vues en perspective et en coupe de la tête d'éclairage du dispositif,
- la figure 6 est une vue éclatée, en perspective, d'un module d'éclairage,
- la figure 7 est une vue en perspective d'une partie d'un dispositif d'éclairage selon une deuxième forme de réalisation, équipé de moyens d'indexage de la poignée,
- la figure 8 est une vue du détail référencée A à la figure 7,
- la figure 9 est une vue en coupe d'une partie du dispositif des figures 7 et 8,
- la figure 10 est une vue en perspective d'une partie d'un dispositif selon une troisième forme de réalisation,
- la figure 11 est une vue en perspective d'une tête d'éclairage d'un dispositif selon une quatrième forme de réalisation,
- les figures 12 et 13 sont des vues éclatées, en perspective, du dispositif d'éclairage de la figure 11,
- les figures 14 et 15 illustrent le principe de diffusion de la tâche lumineuse pour deux lentilles mobiles l'une par rapport à l'autre, conformément à la quatrième forme de réalisation.

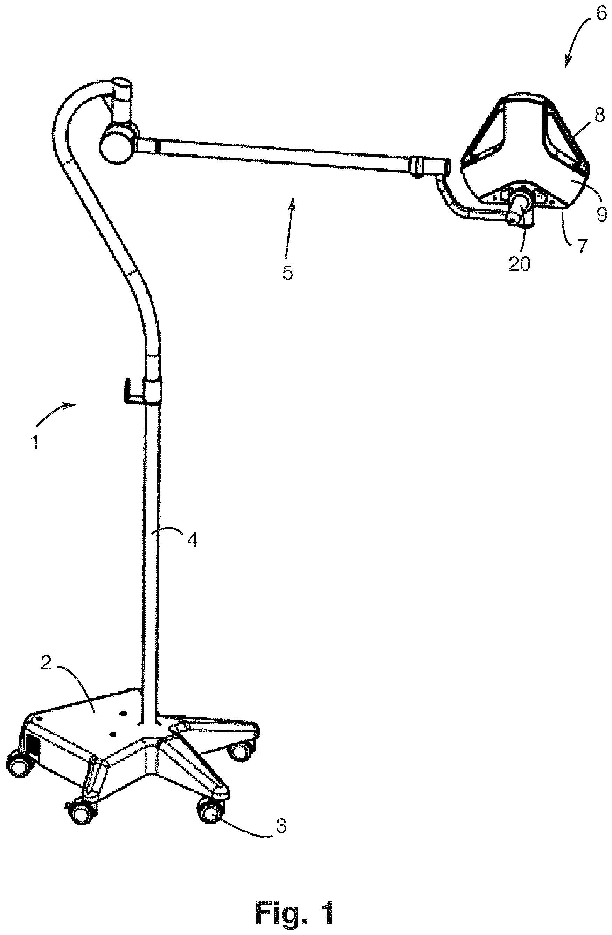

Les figures 1 à 6 représentent un dispositif d'éclairage médial 1, en particulier une lampe scialytique, notamment pour un bloc opératoire.

Le terme "bloc opératoire" inclut également les cas où le dispositif d'éclairage 1 est adapté à un fauteuil dentaire ou à un domaine d'activité similaire : salles d'examen, salles d'urgence, salles de plâtre, unités de soins intensifs, chambres de suture, chambres à induction, néonatologie, pédiatrie, maternité,.... Comme cela est visible à la figure 1, ce dispositif 1 comporte une embase mobile 2 équipée de roues 3, à partir de laquelle s'étend un mat 4, un bras 5 étant articulé sur l'extrémité supérieure du mat 4. Une tête ou coupole d'éclairage 6 est montée sur l'extrémité libre du bras 5. Le bras articulé 5 est formé de plusieurs éléments reliés les uns aux autres par des articulations permettant de déplacer manuellement la tête d'éclairage 6 en tout point de l'espace et d'orienter cette tête 6 dans toutes les directions.

Selon d'autres variantes non représentées, le bras articulé 5 est relié à un point fixe d'un mur ou d'un plafond.

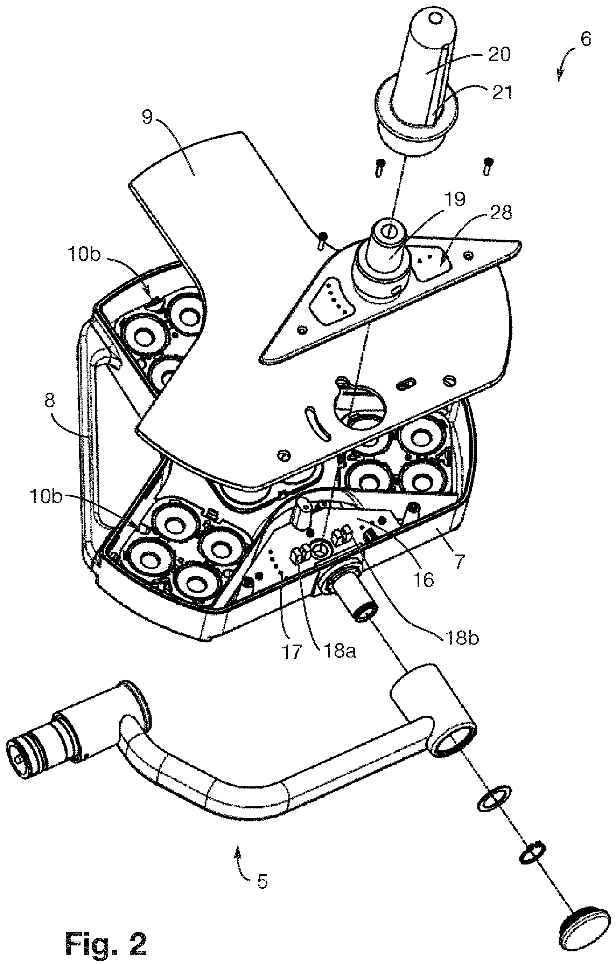

Comme cela est mieux visible aux figures 2 à 5, la tête d'éclairage 6 comporte un corps creux 7 de part et d'autre duquel sont montées des poignées fixes 8. Ces poignées 8 sont fixées à l'intérieur du corps 7 à l'aide de vis, de manière à pouvoir être démontées au besoin. L'ouverture du corps est recouverte d'un capot ou écran transparent de protection 9.

Des modules d'éclairage 10a, 10b sont montés à l'intérieur du corps 7. Plus particulièrement, la tête d'éclairage 6 comporte un module central 10a et quatre modules périphériques 10b, régulièrement répartis autour du module central 10a.

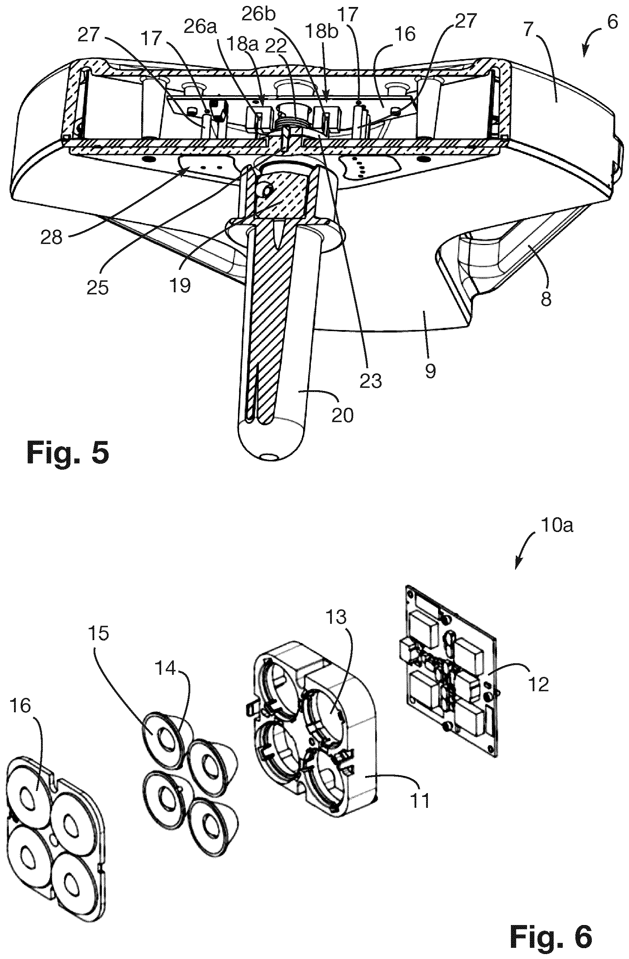

Comme illustré à la figure 6, chaque module 10a, 10b comporte un socle 11 monté sur une carte électronique 12, le socle 11 délimitant quatre logements 13 servant au montage de quatre sources lumineuses, à savoir des diodes électroluminescentes 14, équipées de collimateurs 15.

Les cartes électroniques 12 des modules 10a, 10b permettent de gérer l'allumage des différentes sources lumineuses.

Le module central 10a est équipé d'une plaque pourvue de de collimateurs additionnels 16 de façon à ce que la tâche de lumière formée par le module central 10a à une distance focale déterminée, ici de l'ordre de un mètre, présente une dimension ou un diamètre inférieur à la tâche de lumière formée par chacun des modules périphériques 10b. La tâche de lumière formée par le module central 10a a par exemple un diamètre de l'ordre de 16 cm alors que les tâches de lumière formées par chacun des modules périphériques 10b ont par exemple un diamètre de l'ordre de 22 cm, à la distance focale précitée. L'utilisation de plusieurs modules lumineux 10a, 10b décalés les uns des autres par rapport à l'axe du faisceau émis par le module central 10a permet d'obtenir un effet scialytique tendant à diluer ou réduire l'influence des ombres portées.

Le dispositif 1 comporte également des moyens de commande aptes notamment à commander l'allumage des différents modules 10a, 10b et comprenant une carte électronique de commande 16, montée et fixée dans le corps 7. Ladite carte électronique de commande 16 comporte des diodes électroluminescentes 17 (figures 2 et 5) aptes à fournir une indication quant au mode d'allumage ou de fonctionnement du dispositif 1.

Ladite carte électronique 16 comporte en outre une première et une seconde fourches optiques 18a, 18b, chaque fourche optique comportant un émetteur lumineux et un récepteur situé en regard de l'émetteur et apte à détecter un signal lumineux émis par ledit émetteur.

Un arbre 19a est monté de façon pivotante sur le corps 7. Un support de poignée 19b est solidaire de l'arbre 19a grâce à une vis 19c. Une poignée 20 est montée de façon amovible sur le support 19b. La poignée 20 est ainsi montée pivotante par rapport au corps 7 et peut être séparée de l'arbre 19a afin de pouvoir être changée ou stérilisée par exemple. La poignée 20 peut comporter une zone 21 en saillie longitudinale apte à faciliter la prise en main de la poignée 20 par un opérateur.

L'arbre 19a et la poignée 20 peuvent ainsi pivoter de part et d'autre d'une position angulaire médiane ou centrale. Des moyens de rappel élastiques 22 se présentant sous la forme d'un ressort de torsion tendent à ramener l'arbre 19a et la poignée 20 dans ladite position médiane.

Selon une autre forme de réalisation, le rappel de l'arbre 19a en position médiane peut être sans contact et magnétique avec au moins un aimant permanent monté sur l'arbre 19a, situé en regard d'une denture en matériau ferritique. L'usage de deux aimants permanents, à savoir un aimant mobile et solidaire de l'arbre 19a et un aimant fixe et solidaire du corps 7, agencés en regard l'un de l'autre et en attraction au niveau de la position médiane peut également être envisagé. Enfin l'usage de trois aimants permanents, à savoir un aimant mobile et solidaire de l'arbre 19a et deux aimants fixes en répulsion par rapport au premier, solidaires du corps 7 et placés à chaque extrémité du débattement de l'arbre 19a.

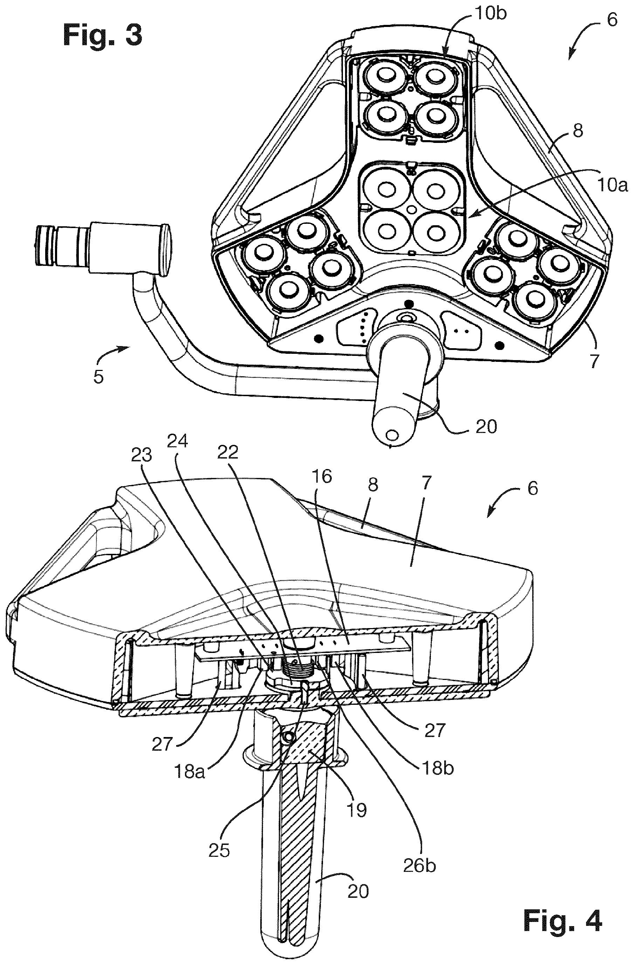

Comme cela est mieux visible à la figure 4, une rondelle 23 comportant une lumière ou une encoche circonférentielle 24 est fixée sur l'arbre 19a, un plot fixe 25 par rapport au corps 7 étant engagé dans la lumière ou l'encoche 24 de façon à pouvoir venir en butée sur les extrémités circonférentielles de la lumière ou de l'encoche 24.

La rotation de l'arbre 19a et de la poignée 20 est ainsi limitée par butée du plot 25 sur lesdites extrémités, entre une première et une seconde positions angulaires extrêmes de la poignée 20 par rapport au corps 7. Le débattement angulaire total de la poignée 20 par rapport au corps 7 est par exemple de l'ordre de 60°, soit 30° de débattement de part et d'autre de la position médiane.

Ladite rondelle 23 porte également une première et une seconde pattes 26a, 26b (figure 5) s'étendant parallèlement à l'axe de l'arbre 19a, en direction respectivement des fourches optiques 18a, 18b. Plus particulièrement, lors du pivotement de la poignée 20, la première patte 26a est apte à former un obstacle venant s'intercaler entre l'émetteur et le récepteur de la première fourche optique 18a, dans une première position angulaire de détection déterminée, la seconde patte 26b étant apte à former un obstacle venant s'intercaler entre l'émetteur et le récepteur de la seconde fourche optique 18b, dans une seconde position angulaire de détection déterminée. La première position angulaire de détection est située à proximité de la première position extrême, et légèrement décalée par rapport à cette position, par exemple d'un angle de 5°. De même, la seconde position de détection est située à proximité de la seconde position extrême, et légèrement décalée par rapport à cette position, par exemple d'un angle de 5°.

Le débattement angulaire entre les deux positions de détection est ainsi par exemple de l'ordre de 50°, soit 25° de part et d'autre de la position médiane.

L'utilisation de fourches optiques 18a, 18b et des pattes 26a, 26b permettent de fournir des indications quant à la position de la poignée 20, sans contact, de manière à augmenter la fiabilité du dispositif 1.

Des guides de lumineux tubulaires 27 (figure 5) sont montés dans le corps 7 et comprennent chacun une première extrémité située en regard et à distance de l'une des diodes électroluminescentes 17 de la carte électronique de commande 16 et une seconde extrémité débouchant au niveau d'une surface d'affichage 28 du corps 7, visible depuis l'extérieur. La lumière générée par chaque diode électroluminescente 17 peut ainsi être guidée jusqu'à la surface d'affichage 28 par le guide lumineux 27 correspondant. Ces guides 27 forment donc des moyens sans contact permettant d'amener une information lumineuse générée à l'intérieur du corps 7 jusque dans une zone 28 située à l'extérieur du corps 7. De tels moyens sans contact permettent ainsi d'augmenter la fiabilité du dispositif 1.

Le dispositif d'éclairage 1 est apte à fonctionner selon les modes suivants :

- un premier mode de fonctionnement dans lequel seul le module central 10a est allumé, de manière à former une tâche de type « spot » de faible diamètre,
- un second mode de fonctionnement dans lequel le module central 10a et l'un des modules périphériques 10b sont allumés simultanément de manière à former une tâche d'intensité et de diamètre plus importants que dans le premier mode,
- un troisième mode de fonctionnement dans lequel le module central 10a et deux des modules périphériques 10b sont allumés simultanément de manière à former une tâche de plus forte intensité que dans le deuxième mode,
- un quatrième mode de fonctionnement dans lequel le module central 10a et les trois modules périphériques 10b sont allumés simultanément de manière une tâche de plus forte intensité que dans le troisième mode,
- un cinquième mode de fonctionnement dans lequel tous les modules 10a, 10b sont éteints.

Le premier mode peut être activé par pivotement de la poignée 20 dans un premier sens de rotation, depuis la position centrale vers la première position, et par maintien de la première position pendant une durée déterminée, par exemple de l'ordre de 1 à 3 secondes.

La poignée 20 retrouve ensuite sa position médiane, par relâchement de la poignée 20, sous l'effet des moyens de rappel élastiques 22 ou magnétiques.

Les second, troisième et quatrième modes peuvent alors être activés successivement par pivotements successifs de la poignée 20 dans le premier sens de rotation, depuis la position centrale vers la première position, sans nécessiter de maintien de la première position pendant une durée déterminée.

Le cinquième mode peut être activé par pivotement de la poignée 20 dans un second sens de rotation de la poignée, opposé au premier sens de rotation, depuis la position centrale de la poignée vers la seconde position, et par maintien de la seconde position pendant une durée déterminée, par exemple de l'ordre de 1 à 3 secondes.

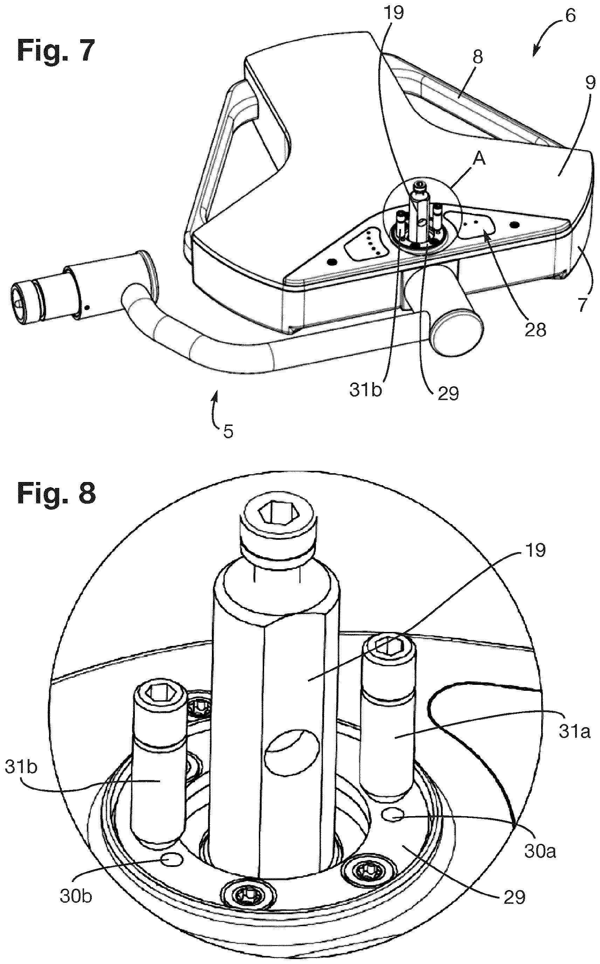

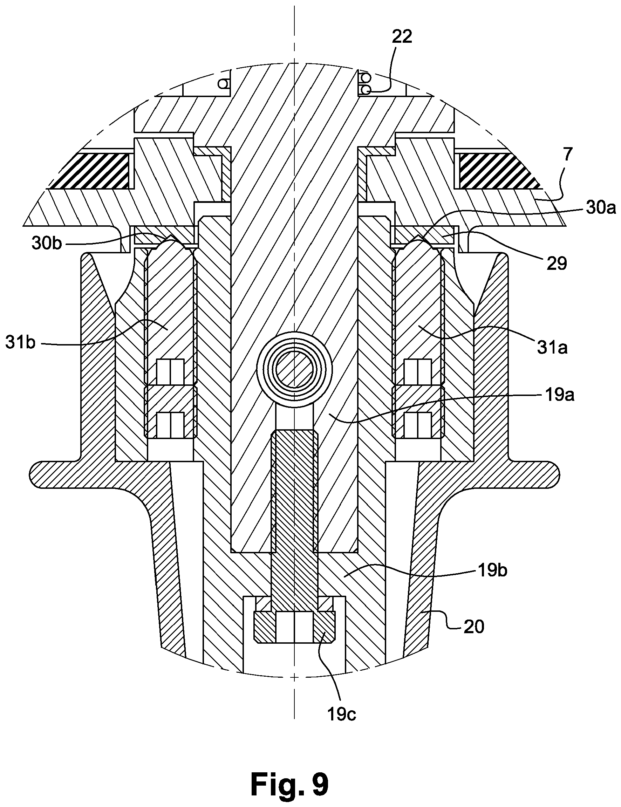

Afin d'améliorer l'ergonomie du dispositif 1, il est possible de prévoir des moyens d'indexage de la position de la poignée, comme cela est représenté aux figures 7 à 9.

Pour cela, une rondelle 29 équipée d'au moins un trou 30a peut être fixée sur le corps 7 et au moins un poussoir à ressort 31a peut être monté sur l'arbre 19a. Il est préférable de doubler le nombre de trous (30a et 30b) et le nombre de poussoirs à ressorts associés (31a et 31b) afin d'augmenter le couple résistant d'indexation et d'annuler le couple parasite sur l'arbre 19a. Chaque poussoir 31a, 31b comporte une bille soumise à l'action d'un organe élastique de rappel, la bille du premier poussoir 31a étant apte à être engagée dans le premier trou 30a, la bille du second poussoir 31b étant apte à être engagée dans le second trou 30b simultanément à l'engagement de la bille du premier poussoir 31a. Le but de l'indexation est de générer un couple résistant avant les positions de détection afin de réduire le risque de contrôle intempestif lors du positionnement de la tête d'éclairage dans l'espace par un opérateur.

Les premier et second poussoirs 31a, 31b sont ainsi aptes à exercer un couple résistant sur la poignée 30 dans au moins une position déterminée de la poignée 30, par exemple dans la position angulaire médiane de la poignée 30. De tels moyens d'indexage peuvent également être utilisés pour indexer d'autres positions, telles que par exemple juste avant la première et la seconde positions de détection.

Selon une autre forme de réalisation, l'indexation en position peut être sans contact et magnétique avec au moins un aimant permanent monté sur l'arbre 19a, situé en regard d'une denture en matériau ferritique. L'usage de deux aimants permanents, à savoir un aimant mobile et solidaire de l'arbre 19a et un aimant fixe et solidaire du corps 7, agencés en regard l'un de l'autre et en attraction au niveau de la position médiane peut également être envisagé. Enfin l'usage de trois aimants permanents, à savoir un aimant mobile et solidaire de l'arbre 19a et deux aimants fixes en répulsion par rapport au premier, solidaires du corps 7 et placés à chaque extrémité du débattement de l'arbre 19a.

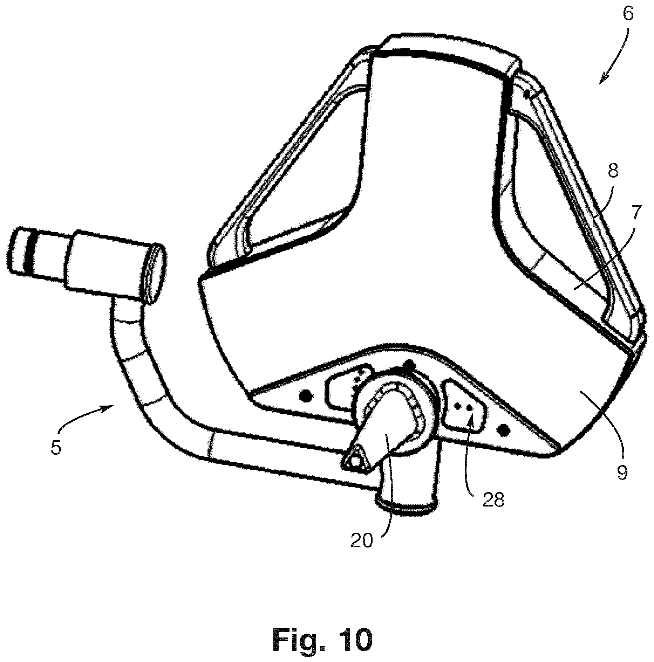

La figure 10 illustre un dispositif selon une autre forme de réalisation dans laquelle la poignée 20 présente une section polygonale, par exemple triangulaire, dans un plan perpendiculaire à l'axe de la poignée 20, de façon à faciliter la prise en main par un opérateur.

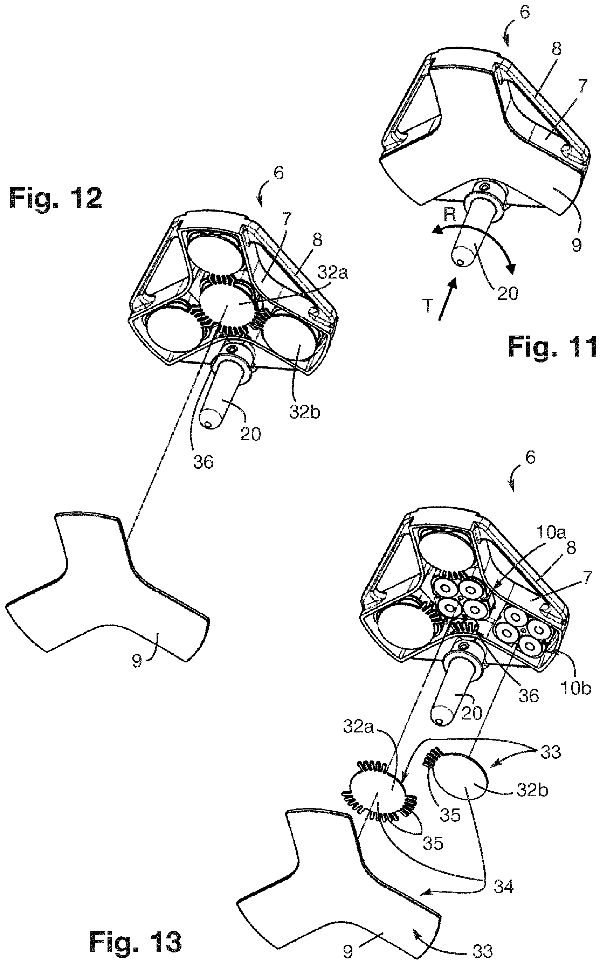

Les figures 11 à 13 illustrent un dispositif selon une autre forme de réalisation selon une autre de réalisation de l'invention, dans laquelle chaque module 10a, 10b est équipé d'une lentille mobile 32a, 32b par rapport à l'écran de protection 9.

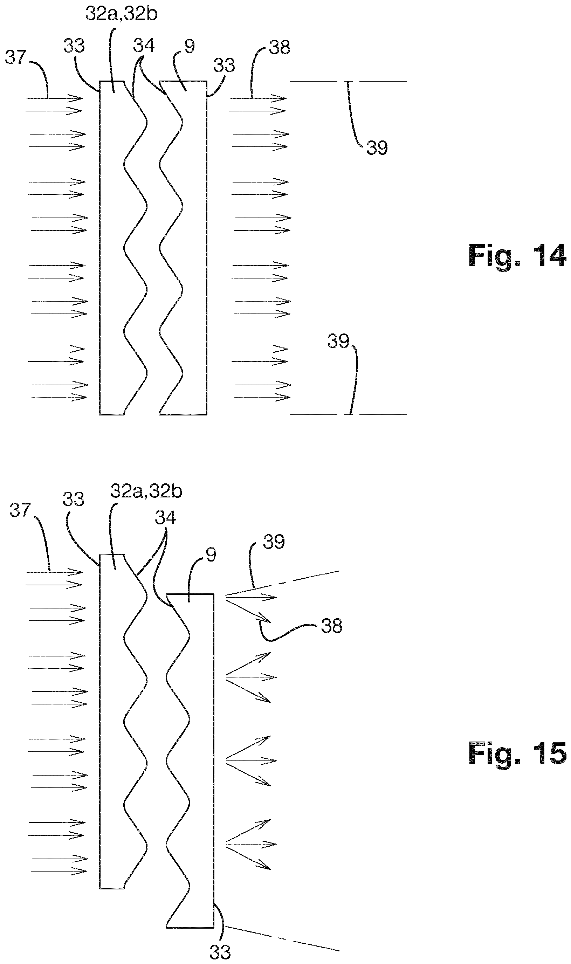

Les lentilles mobiles 32a, 32b comportent une surface plane 33 et une surface ondulée 34 présentant des creux et des saillies (figures 14, 15). Par ailleurs, l'écran de protection fixe 9 comporte une surface plane 33 et une surface ondulée 34.

Les surfaces ondulées 34 des lentilles, 32a, 32b et de l'écran 9 sont agencées en regard l'une de l'autre, de façon à faire converger ou diverger les rayons lumineux qui les traversent en fonction de leurs positions respectives.

Les alternances de zones en creux et en saillie sont orientées dans deux directions, respectivement en direction circonférentielle et en direction radiale.

Les lentilles mobiles 32a, 32b se présentent sous la forme de disques présentant des moyens d'engrenage 35. La poignée 20 est également couplée à des moyens d'engrenage 36 aptes à engrener avec la lentille mobile 32a du module central 10a qui, elle-même, engrène avec toutes les lentilles mobiles 32b des modules périphériques 10b. Le pivotement de la poignée 20 permet donc, dans un mode de fonctionnement particulier décrit ci-après, de faire pivoter simultanément toutes les lentilles mobiles 32a, 32b par rapport aux modules fixes 10a, 10b ou par rapport à l'écran de protection 9 fixe.

Le principe général des moyens de diffusion à l'aide de deux plaques mobiles l'une par rapport à l'autre est connu du document US 5 775 799. L'invention permet d'adapter ce principe général au cas des modules précités.

Ce principe va maintenant être décrit en référence aux figures 14 et 15 représentent schématiquement une lentille mobile 32a, 32b et l'écran de protection fixe 9, dans deux positions différentes de la lentille mobile 32a, 32b.

En position de non diffusion, représentée à la figure 14, les lentilles 32a, 32b et l'écran 9 sont positionnés de telle manière que les creux de la lentille mobile 32a, 32b sont disposés en regard des saillies de l'écran fixe 9 et inversement. Les pas des creux et des saillies sont identiques d'une lentille à l'autre, aussi bien en direction circonférentielle qu'en direction radiale.

Dans cette position, les effets des deux lentilles s'annulent et le faisceau de lumière collimaté 37 provenant des diodes électroluminescentes émerge de l'écran 9 encore sensiblement collimaté (voir flèches 38), c'est-à-dire de manière non diffuse. Ceci est représenté par les lignes de référence 39 sur la figure 14.

Pour la diffusion de la lumière, les lentilles, 32a, 32b et l'écran 9 sont positionnés de telle manière que les creux de la lentille mobile 32a, 32b sont disposés en regard des creux de l'écran fixe 9.

Dans cette position, les effets de divergence des deux lentilles se cumulent et le faisceau de lumière émerge de l'écran 9 de façon très diffuse. Ceci est représenté schématiquement par les lignes de référence 39 sur la figure 15.

Des positions intermédiaires de la lentille mobile 32a, 32b vis-à-vis de l'écran fixe 9 permettent d'obtenir une diffusion plus moins ou moins importante du flux de lumière, et donc une tâche lumineuse plus ou moins grande.

La surface ondulée de la chaque lentille est une nappe sinusoïdale qui peut être exprimée en coordonnées sphériques sous la forme mathématique suivante :

$$R * \left[ 1 + K * \cos\left( \varphi_{min} + D_\varphi * \varphi \right) * \cos\left( P_\varphi * \varphi * \frac{\cos(\varphi_{min})}{\cos\left( \varphi_{min} + \dfrac{D_\varphi * \varphi}{2} \right)} \right) * \cos(P_\theta * \theta) \right]$$

dans laquelle :

θ est l'angle de longitude,

φ est l'angle de latitude,
R est le rayon de courbure (galbe) des lentilles,
K est l'amplitude relative de la nappe sinusoïdale,
Pθ est la période selon l'angle θ,
Pφ est la période selon l'angle φ,
Dφ est la différence de latitude entre les bords longitudinaux de la surface ondulée,
φmin est l'angle de latitude minimal.

En ajustant la formule mathématique du paragraphe précédent aux paramètres dimensionnels du module d'éclairage, on obtient la surface optique ondulée recherchée, qui est ensuite appliquée sur les surfaces 34 en regard des lentilles 32a, 32b et de l'écran 9.

La période des ondulations dépend préférentiellement du diamètre apparent des sources lumineuses, c'est-à-dire des diodes électroluminescentes 14. Il est en effet préférable qu'une demi-période d'ondulation (bosse ou creux) soit entièrement contenue dans le diamètre apparent d'une source lumineuse.

Préférentiellement, le diamètre apparent de la source lumineuse est beaucoup plus grand qu'une demi-période de la surface ondulée 34. Cette période influe également sur l'amplitude de déplacement, entre les lentilles 32a, 32b et l'écran 9 nécessaire pour le passage de la position de non-diffusion (figure 14) à la position de diffusion (figure 15).

On notera que l'amplitude des ondulations est sensiblement constante, quel que soit l'angle de longitude. Afin d'augmenter le diamètre de la tâche de lumière, il est notamment possible d'augmenter l'amplitude des ondulations. Si l'on cherche à obtenir une trop grande tâche de lumière, c'est-à-dire une trop grande diffusion du flux lumineux émis par les diodes électroluminescentes 14, on observe que la tâche comporte en son centre une zone dont l'intensité lumineuse est plus faible, appelée zone « d'ombre ».

Afin d'éviter un tel phénomène, une variante de réalisation consiste à faire varier l'amplitude des ondulations en fonction de la longitude de sorte que la surface ondulée 34 présente une zone très diffusante, permettant d'obtenir une tâche large avec une zone d'ombre centrale, et une zone moins diffusante, permettant d'obtenir une tâche de diamètre plus faible éclairant la zone d'ombre. D'un point de vue mathématique, cette opération consiste à remplacer l'amplitude relative K dans la formule précitée par une fonction $K(\theta)$ qui dépend de la longitude $\theta$ de manière non linéaire.

On définit par exemple par diamètre de la tâche lumineuse, le diamètre de la zone dont l'intensité lumineuse est supérieure à 10% de l'intensité maximale.

On pourrait élargir encore davantage la tâche lumineuse, mais cela n'est pas utile pour les applications médicales usuelles et l'usinage des ondulations serait dans ce cas plus complexe à réaliser.

Dans cette forme de réalisation, la poignée 20 est mobile en translation le long de son axe, comme illustré par la flèche T à la figure 11, entre deux positions axiales. Une première position permet de sélectionner l'un des cinq modes de fonctionnement décrit précédemment, une seconde position permettant de faire varier la position des lentilles mobiles 32a, 32b par rapport aux lentilles fixes 15, 16, par pivotement de la poignée 20 (illustré par la flèche R à la figure 11).

Le pivotement de la poignée 20, et donc le déplacement des lentilles mobiles 32a, 32b par rapport à l'écran fixe 9, sont limités par la présence des butées 24, 25.

## Revendications

1. Dispositif d'éclairage médical (1), notamment pour un bloc opératoire, comportant une tête d'éclairage (6) comprenant une première source lumineuse (14, 10a) et au moins une deuxième source lumineuse (14, 10b), décalée latéralement de la première source lumineuse (14, 10a) par rapport à la direction d'éclairage de la première source lumineuse (14, 10a), des moyens de commande (16, 12) aptes à allumer la première source lumineuse (14, 10a) dans un premier mode d'éclairage et aptes à allumer la première source lumineuse (14, 10a) et la deuxième source lumineuse (14, 10b) dans au moins un deuxième mode d'éclairage, la tête d'éclairage (6) comportant un corps (7) dans lequel sont montées les sources lumineuses (14, 10a, 10b), une poignée (20) s'étendant selon un axe, montée sur le corps (7) et mobile en rotation par rapport au corps (7) autour de l'axe de ladite poignée (20), des moyens de commande (16, 12) situés dans le corps (7) et aptes à commander l'arrêt ou l'allumage des sources lumineuses (14, 10a, 10b), le passage d'un mode d'éclairage à l'autre étant obtenu par pivotement de la poignée (20) autour de son axe, la course angulaire de la poignée (20) étant limitée par deux butées (24, 25) définissant deux positions extrêmes opposées de la poignée (20), le dispositif comportant en outre des moyens de détection (18a, 18b, 26a, 26b) aptes à détecter respectivement une première et une seconde positions angulaires de détection de la poignée (20), décalées angulairement respectivement par rapport auxdites positions extrêmes.

2. Dispositif (1) selon la revendication 1, **caractérisé en ce qu'**il comprend au moins une troisième source lumineuse, décalée latéralement de la première source lumineuse (14, 10a) par rapport à la direction d'éclairage de la première source lumineuse (14, 10a), les moyens de commande (16, 12) étant aptes à allumer les première, deuxième et troisième sources lumineuse (14, 10a, 10b) dans un troisième mode d'éclairage.

3. Dispositif (1) selon la revendication 1 ou 2, comprenant au moins une quatrième source lumineuse, décalée latéralement de la première source lumineuse (14, 10a) par rapport à la direction d'éclairage de la première source lumineuse (14, 10a), les moyens de commande (16, 12) étant aptes à allumer les première, deuxième, troisième et quatrième sources lumineuse (14, 10a, 10b) dans un quatrième mode d'éclairage.

4. Dispositif (1) selon la revendication 2 ou 3, **caractérisé en ce que** la première source lumineuse forme une source lumineuse centrale, les deuxième, troisième et/ou quatrième sources lumineuses formant des sources lumineuses périphériques régulièrement réparties autour de la première source lumineuse.

5. Dispositif (1) selon l'une des revendications 1 à 4, **caractérisé en ce que** les différentes sources lumineuses sont aptes à générer respectivement des tâches lumineuses superposées, au moins en partie, à une distance focale déterminée.

6. Dispositif (1) selon l'une des revendications 1 à 5, **caractérisé en ce qu'**il comporte des moyens d'indexage (30a, 30b, 31a, 31b), aptes à exercer un couple résistant sur la poignée (20), dans au moins une position angulaire déterminée de la poignée (20), de façon à indexer ladite position déterminée.

7. Dispositif (1) selon la revendication 6, **caractérisé en ce que** les moyens d'indexage sont aptes à indexer des positions situées entre les première et seconde positions angulaires de détection.

8. Dispositif (1) selon l'une des revendications 1 à 7, caractérisé en en ce que les moyens de détection comportent au moins une première partie (26a, 26b) couplée en rotation par rapport à la poignée (20) et au moins une seconde partie (18a, 18b) couplée en rotation par rapport au corps (7) et apte à fournir aux moyens de commande (16, 12) un signal qui est représentatif de la position angulaire la poignée (20) par rapport au corps (7), la seconde partie (18a, 18b) étant apte à détecter la position angulaire de la première partie (26a, 26b) sans contact, ou inversement.

**Patentansprüche**

1. Medizinische Beleuchtungsvorrichtung (1), insbesondere für einen OP-Saal, welche einen Beleuchtungskopf (6) mit einer ersten Lichtquelle (14, 10a) aufweist und mindestens einer zweiten Lichtquelle (14, 10b), die im Verhältnis zur ersten Lichtquelle (14, 10a) relativ zur Beleuchtungsrichtung der ersten Lichtquelle (14, 10a) seitlich versetzt ist, Steuermittel (16, 12), die in der Lage sind, die erste Lichtquelle (14, 10a) in einer ersten Beleuchtungsart einzuschalten und in der Lage sind, die erste Lichtquelle (14, 10a) und die zweite Lichtquelle (14, 10b) in mindestens einer zweiten Beleuchtungsart einzuschalten, wobei der Beleuchtungskopf (6) einen Körper (7) umfasst, in den die Lichtquellen (14, 10a, 10) montiert sind, einen Griff (20), der sich an einer Achse entlang erstreckt, auf den Körper (7) montiert ist und in Bezug zum Körper (7) um die Achse besagten Griffs (20) drehbar ist, am Körper (7) befindliche Steuerungsmittel (16, 12), die das Ausschalten und Einschalten der Lichtquellen (14, 10a, 10b) steuern können, wobei der Wechsel von der ersten Beleuchtungsart zur zweiten Beleuchtungsart durch Drehen des Griffs (20) erfolgt, wobei der Schwenkwinkel des Griffs (20) durch zwei Anschläge (24, 25) begrenzt wird, welche zwei einander gegenüberliegende Endstellungen des Griffs (20) abgrenzen, wobei die Vorrichtung darüber hinaus Detektionsmittel (18a, 18b, 26a, 26b) umfasst, die in der Lage sind, jeweils eine erste und eine zweite Winkeldetektionsposition des Griffs (20) zu erkennen, die jeweils im Verhältnis zu den besagten Endpositionen winkelförmig versetzt sind.

2. Vorrichtung (1) nach Anspruch 1, **dadurch gekennzeichnet, dass** sie mindestens eine dritte Lichtquelle aufweist, die relativ zur Beleuchtungsrichtung der ersten Lichtquelle (14, 10a) seitlich zur ersten Lichtquelle (14, 10a) versetzt ist, Steuerungsmittel (16, 12), die die erste, die zweite und die dritte Lichtquelle (14, 10a, 10b) in einer dritten Beleuchtungsart einschalten können.

3. Vorrichtung (1) nach Anspruch 1 oder 2, **dadurch gekennzeichnet, dass** sie mindestens eine vierte Lichtquelle aufweist, die relativ zur Beleuchtungsrichtung der ersten Lichtquelle (14, 10a) seitlich zur ersten Lichtquelle (14, 10a) versetzt ist, wobei die Steuerungsmittel (16, 12), die erste, die zweite, dritte und vierte Lichtquelle (14, 10a, 10b) in einer vierten Beleuchtungsart einschalten können.

4. Vorrichtung (1) nach Anspruch 2 oder 3, **dadurch gekennzeichnet, dass** die erste Lichtquelle eine mittlere Lichtquelle bildet, und die zweite, dritte und/oder vierte Lichtquelle Lichtquellen am Rand bilden, die gleichmäßig um die erste Lichtquelle angeordnet sind.

5. Vorrichtung (1) nach einem der Ansprüche 1 bis 4, **dadurch gekennzeichnet, dass** die Lichtquellen in der Lage sind, in einer bestimmten Brennweite Lichtflecken zu erzeugen, die zumindest teilweise überlagert sind.

6. Vorrichtung (1) nach einem der Ansprüche 1 bis 5, **dadurch gekennzeichnet, dass** sie Arretierungsmittel (30a, 30b, 31a, 31b) umfasst, die in der Lage sind, in mindestens einer bestimmten Winkelposition des Griffs (20) ein Gegenmoment am Griff (20) zu erzeugen, so dass besagte bestimmte Position arretiert wird.

7. Vorrichtung (1) nach Anspruch 6, **dadurch gekennzeichnet, dass** die Arretierungsmittel in der Lage sind, Positionen zwischen der ersten und der zweiten Winkeldetektionsposition zu arretieren.

8. Vorrichtung (1) nach einem der Ansprüche 1 bis 7, **dadurch gekennzeichnet, dass** die Detektionsmittel mindestens einen ersten Teil (26a, 26b) umfassen, der relativ zum Griff (20) drehbar gelagert ist, und mindestens einen zweiten Teil (18a, 18b), der relativ zum Körper (7) drehbar gelagert ist und den Steuerungsmitteln (16, 12) ein Signal senden kann, das repräsentativ für die Winkelposition des Griffs (20) relativ zum Körper (7) ist, wobei der zweite Teil (18a, 18b) in der Lage ist, die Winkelposition des ersten Teils (26a, 26b) kontaktlos zu erkennen, oder umgekehrt.

**Claims**

1. A medical lighting device (1), especially for an operating theater, including a lighting head (6) comprising a first light source (14, 10a) and at least one second light source (14, 10b), laterally offset relative to the first light source (14, 10a) relative to the lighting direction of the first light source (14, 10a), control means (16, 12) adapted to turn on the first light source (14, 10a) in a first lighting mode and adapted to turn on the first light source (14, 10a) and the second light source (14, 10b) in at least one second lighting mode, with the lighting head (6) comprising a body (7) wherein the light sources (14, 10a, 10b) are mounted, one handle (20) extending along an axis, mounted on the body (7) and rotatable relative to the body (7) about the axis of said handle (20), control means (16, 12) located in the body (7) and adapted to turn on or off the light sources (14, 10a, 10b), with the switching from one lighting mode to another being obtained by pivoting the handle (20) about the axis thereof, with the angular travel of the handle (20) being limited by two stops (24, 25) defining two opposed extreme positions of the handle (20), with the device further including detection means (18a, 18b, 26a, 26b) adapted to respectively detect a first and a second angular positions of detection of the handle (20), angularly offset relative to said extreme positions.

2. A device (1) according to claim 1, **characterized in that** it comprises at least one third light source, laterally offset relative to the first light source (14, 10a), relative to the lighting direction of the first light source (14, 10a), with the control means (16, 12) being adapted to turn on the first, second and third light sources (14, 10a, 10b) in a third lighting mode.

3. A device (1) according to claim 1 or 2, comprising at least one fourth light source, laterally offset relative to the first light source (14, 10a), relative to the lighting direction of the first light source (14, 10a), with the control means (16, 12) being adapted to turn on the first, second, third and fourth light sources (14, 10a, 10b) in a fourth lighting mode.

4. A device (1) according to claim 2 or 3, **characterized in that** the first light source forms a central light source, with the second, third and/or fourth light sources forming peripheral light sources regularly distributed around the first light source.

5. A device (1) according to one of claims 1 to 4, **characterized in that** the various light sources are adapted to respectively generate at least partially superimposed light spots, at a determined focal length.

6. A device (1) according to one of claims 1 to 5, **characterized in that** it includes indexing means (30a, 30b, 31a, 31b), which is adapted to exert a resisting torque onto the handle (20) in at least one determined angular position of the handle (20), so as to index said determined position.

7. A device (1) according to claim 6, **characterized in that** the indexing means is adapted to index positions between the first and second angular detection positions.

8. A device (1) according to one of claims 1 to 7, **characterized in that** the detection means includes at least one first portion (26a, 26b) rotatably coupled relative to the handle (20) and at least one second portion (18a, 18b) rotatably coupled relative to the body (7) and which is adapted to supply the control means (16, 12) with a signal which is representative of the angular position of the handle (20) relative to the body (7), with the second portion (18a, 18b) being adapted to detect the angular position of the first portion (26a, 26B) with no contact, or vice versa.

**Fig. 1**

**Fig. 2**

**Fig. 3**

**Fig. 4**

**Fig. 5**

**Fig. 6**

## Fig. 7

## Fig. 8

**Fig. 9**

**Fig. 10**

Fig. 12

Fig. 11

Fig. 13

**Fig. 14**

**Fig. 15**

## RÉFÉRENCES CITÉES DANS LA DESCRIPTION

### Documents brevets cités dans la description

- EP 1722157 A
- EP 2065634 A
- US 5775799 A